# EUROPÄISCHE PATENTSCHRIFT

(11) **EP 3 573 792 B1**
(45) Veröffentlichungstag und Bekanntmachung des Hinweises auf die Patenterteilung: **14.09.2022**
(21) Anmeldenummer: 18702652.1
(22) Anmeldetag: 29.01.2018
(51) Int. Cl.: B25J 9/16, B25J 13/00, A61B 34/30, A61B 34/35, A61B 17/00

(54) **OPERATIONS-ASSISTENZ-SYSTEM UND VERFAHREN ZUR ERZEUGUNG VON STEUERSIGNALEN ZUR SPRACHSTEUERUNG EINER MOTORISCH GESTEUERT BEWEGBAREN ROBOTERKINEMATIK EINES DERARTIGEN OPERATIONS-ASSISTENZ-SYSTEMS**
OPERATION-ASSISTANCE SYSTEM AND METHOD FOR GENERATING CONTROL SIGNALS FOR THE VOICE CONTROL OF AN OPERATION-ASSISTANCE SYSTEM KINEMATIC ROBOT THAT CAN BE MOVED IN A MOTOR-CONTROLLED MANNER
SYSTÈME D'ASSISTANCE OPÉRATOIRE ET PROCÉDÉ PERMETTANT DE PRODUIRE DES SIGNAUX DE COMMANDE POUR LA COMMANDE VOCALE D'UNE CINÉMATIQUE DE ROBOT DÉPLAÇABLE MOTORISÉE D'UN SYSTÈME D'ASSISTANCE OPÉRATOIRE

(30) Priorität: 30.01.2017 DE 102017101782
(43) Veröffentlichungstag der Anmeldung: 04.12.2019
(73) Patentinhaber: Aktormed GmbH, 93092 Barbing (DE)
(72) Erfinder: GEIGER, Robert, 94526 Metten (DE)
(74) Vertreter: Glück Kritzenberger Patentanwälte PartGmbB
(86) Internationale Anmeldenummer: PCT/EP2018/052113
(87) Internationale Veröffentlichungsnummer: WO 2018/138325

(56) Entgegenhaltungen:
- WO-A1-96/09587
- US-A1- 2013 096 575
- US-A1- 2015 088 060
- US-B1- 6 591 239

## Beschreibung

Die Erfindung betrifft ein Operations-Assistenz-System gemäß dem Oberbegriff des Patentanspruches 1 und ein Verfahren zur Erzeugung von Steuersignalen zur Sprachsteuerung einer motorisch gesteuert bewegbaren Roboterkinematik eines Operations-Assistenz-Systems gemäß dem Oberbegriff des Patentanspruches Operations-Assistenz-Systeme, insbesondere zur Unterstützung von medizinischen Eingriffen oder Operationen, insbesondere minimal-invasiven Operationen sind hinlänglich bekannt.

Derartige Operations-Assistenz-Systeme werden häufig zur Führung von medizinischen Hilfsinstrumenten, wie z.B. Kamerasystemen, insbesondere so genannten Endoskopkameras verwendet. Aus der DE 10 2007 019363 A1 ist beispielsweise ein Operations-Assistenz-System bekannt, mittels dem beispielsweise ein eine Kameraeinheit aufweisendes Endoskop bzw. eine Endoskopkamera gesteuert geführt wird. Das Operations-Assistenz-System weist hierzu eine gesteuert antreibbare Roboterkinematik auf, mittels der eine an einer Instrumentenhalterung aufgenommene Endoskopkamera im dreidimensionalen Raum, insbesondere im Operationsraum gesteuert bewegbar ist. Die Roboterkinematik umfasst beispielsweise wenigstens eine Tragsäule, wenigstens zwei Roboterarme und wenigstens einen die Instrumentenhalterung aufnehmenden Instrumententräger.

Aus der DE 10 2008 016 146 B4 ist ferner ein Verfahren zur Führung eines medizinischen Hilfsinstrumentes, insbesondere einer Endoskopkamera mittels eines derartigen Operations-Assistenz-Systems bekannt, und zwar abhängig von der manuellen Betätigung zumindest einer Funktionstaste eines Bedienelements. Das medizinische Hilfsinstrument ist dabei an einem eine Instrumentenhalterung aufweisenden Armsystem des Operations-Assistenz-Systems befestigt und die Spitze des chirurgischen Hilfsinstruments ist mittels des Armsystems in einem kartesischen Patientenkoordinatensystem gesteuert bewegbar, wobei zumindest eine der drei Raumachsen des kartesischen Patientenkoordinatensystem durch die das chirurgische Hilfsinstrument aufnehmende Operationsöffnung bzw. den Trokarpunkt verläuft. Nachteilig ist zur Steuerung der Führung des medizinischen Hilfsinstrumentes, insbesondere einer Endoskopkamera ein mit einem Computersystem verbundenes Bedienteil beispielsweise in der Form eines Fußschalters, Joysticks oder Handbedienteils erforderlich, welches der Operateur zusätzlich zur Führung eines medizinischen Operationsinstruments, mit dem er den operativen Eingriff durchführt, zu bedienen hat. Hierbei ist es erforderlich, dass der Operateur, um eine korrekte Führung des medizinischen Hilfsinstrumentes im Operationsraum zu erreichen, die entsprechenden Funktionstasten des Bedienteils manuell zu betätigen hat, und zwar abhängig von dem ihm mittels einer Monitoreinheit angezeigten Aufnahme des Operationsraumes.

Auch sind bereits sprachgesteuerte Steuersysteme für derartige Operations-Assistenz-Systeme bekannt, bei denen der Operateur über Sprachbefehle eine entsprechende Führung des Hilfsinstruments über die Roboterkinematik steuern kann. Siehe zum Beispiel Dokument WO 96/09587 A1.

Nachteilig ist der Funktionsumfang derartiger Sprachsteuerungen häufig beschränkt und aufgrund dessen das gezielte Anfahren einer Zielposition wenig bedienerfreundlich ausführbar ist.

Ausgehend davon ist es Aufgabe der Erfindung, ein Operations-Assistenz-System, insbesondere auch für medizinische Interventionen oder Operationen sowie ein zugehöriges Verfahren zur Erzeugung von Steuersignalen zur Sprachsteuerung der Roboterkinematik eines derartigen Operations-Assistenz-Systems aufzuzeigen, welches dem Operateur eine genauere und bedienerfreundliche Sprachsteuerung der Führung des medizinischen Hilfsinstruments über das Operations-Assistenz-System bei der Durchführung eines operativen Eingriffs ermöglicht. Die Aufgabe wird durch ein Operations-Assistenz-System mit den Merkmalen des Oberbegriffes des Patentanspruches 1 durch dessen kennzeichnende Merkmale gelöst. Ferner wird die Aufgabe ausgehend von einem Verfahren mit den Merkmalen des Oberbegriffes des Patentanspruches 6 durch dessen kennzeichnende Merkmal gelöst.

Ein wesentlicher Aspekt des erfindungsgemäßen Operations-Assistenz-Systems ist darin zu sehen, dass die Sprachsteuerroutine zur Erfassung und Auswertung von aus zumindest einem ersten und zweiten Sprachbefehl bestehenden Sprachbefehlskombinationen ausgebildet ist, wobei dem ersten Sprachbefehl eine Richtungs- oder Geschwindigkeitsinformation und dem zweiten Sprachbefehl eine Betragsinformation in einem Bezugskoordinatensystem zugeordnet sind, dass die zeitlich unmittelbar aufeinander folgenden ersten und zweiten Sprachbefehle von der Sprachsteuerroutine innerhalb einer vorgegebenen Zeitdauer erfasst und ausgewertet werden, und dass abhängig von der erfassten Richtungs- oder Geschwindigkeitsinformation und der erfassten Betragsinformation der Sprachbefehlskombination zumindest ein zugeordnetes Steuersignal ermittelt wird, über welches zumindest die Bewegung des medizinischen Hilfsinstrumentes richtungs- oder geschwindigkeitsmäßig und betragsmäßig gesteuert wird wobei die Richtungsinformation die Bewegungsrichtung des Hilfsinstrumentes in dem kartesischen Bezugskoordinatensystem und die Betragsinformation die Weglänge bzw. Wegstrecke entlang der Bewegungsrichtung in dem kartesischen Bezugskoordinatensystem angibt. Besonders vorteilhaft kann der Operateur über die Eingabe einer Sprachbefehlskombination die Bewegungsrichtung oder die Geschwindigkeit und den zugehörigen Betrag gleichzeitig einstellen und damit die Bewegung des Hilfsinstrumentes wesentlich genauer steuern, wobei besonders vorteilhaft die Richtungsinformation die Bewegungsrichtung des Hilfsinstrumentes in dem kartesischen Bezugskoordinatensystem und die Betragsinformation die Weglänge bzw. Wegstrecke entlang der Bewegungsrichtung in dem kartesischen Bezugskoordinatensystem angeben.

Mittels an sich einfacher Sprachbefehle können damit komplexere Steuerungsaufgaben realisiert werden, da der Operateur durch die erfindungsgemäße Kombination der einfachen Sprachbefehle einen komplexere Steuerungsbefehl selbst und anwendungsspezifisch erstellen kann, ohne weitere Steuerbefehle erlernen zu müssen.

Betragsinformation Erfindungsgemäß ist die Sprachsteuerroutine zur Erfassung und Auswertung von zeitlich unmittelbar aufeinander folgenden ersten und zweiten Sprachbefehlen innerhalb einer vorgegebenen Zeitdauer eingerichtet. Wird das vorgegebene Zeitintervall überschritten, so wird eine Folge von zwei aufeinanderfolgenden Sprachbefehlen nicht mehr als Sprachbefehlskombination erkannt, sondern als jeweils einzelne Sprachbefehle, die unabhängig voneinander zu erfassen und auszuwerten sind.

Weiterhin vorteilhaft umfassen der erste und zweite Sprachbefehl ein oder mehrere Wörter. Dadurch besteht die Möglichkeit intuitiv beschreiben Wörter und Wortkombinationen für Sprachsteuerung zu verwenden.

Die Sprachsteuerung mittels der Sprachsteuerroutine ist in einer bevorzugten Ausführungsvariante ausschließlich bei Betätigung eines Aktivierungselementes, vorzugsweise eines Aktivierungsschalters oder -tasters durch den Operateur oder bei Vorliegen eines Aktivierungssprachbefehls aktiv.

Weiterhin vorteilhaft kann mittels eines dritten Sprachbefehls die Istposition des Hilfsinstrumentes bezogen auf das Bezugskoordinatensystem gespeichert werden und mittels eines vierten Sprachbefehls zu einem späteren Zeitpunkt wieder gezielt angefahren werden.

Die Ausdrucke "näherungsweise", "im Wesentlichen" oder "etwa" bedeuten im Sinne der Erfindung Abweichungen vom jeweils exakten Wert um +/- 10%, bevorzugt um +/- 5% und/oder Abweichungen in Form von für die Funktion unbedeutenden Änderungen.

Die Erfindung wird im Folgenden anhand der Figuren an Ausführungsbeispielen näher erläutert. Es zeigen:
- Fig. 1: eine schematische Seitenansicht ein Operations-Assistenz-Systems,
- Fig. 2: eine schematische Schnittansicht durch einen Patientenkörper mit im Operationsraum zumindest abschnittsweise aufgenommener Endoskopkamera sowie einem medizinischen Operationsinstrument,

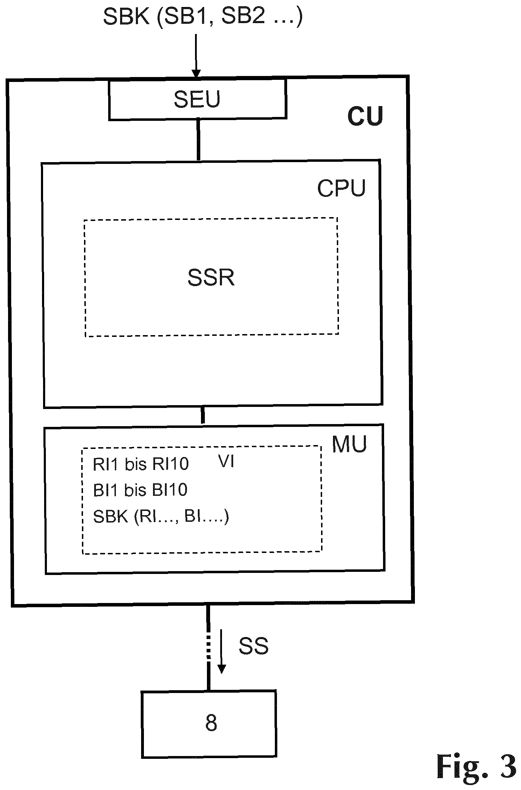

- Fig. 3: ein schematisches Blockschaltbild einer erfindungsgemäßen Sensoreinheit zur Erfassung der Bewegungsdaten des medizinischen Operationsinstruments, und
- Fig. 4a, b: schematische Draufsichten auf ein Diagramm in der x-y-Ebene bzw. x-z-Ebene eines kartesischen Bezugskoordinatensystems umfassend unterschiedliche Bewegungs- und Betragsinformationen.

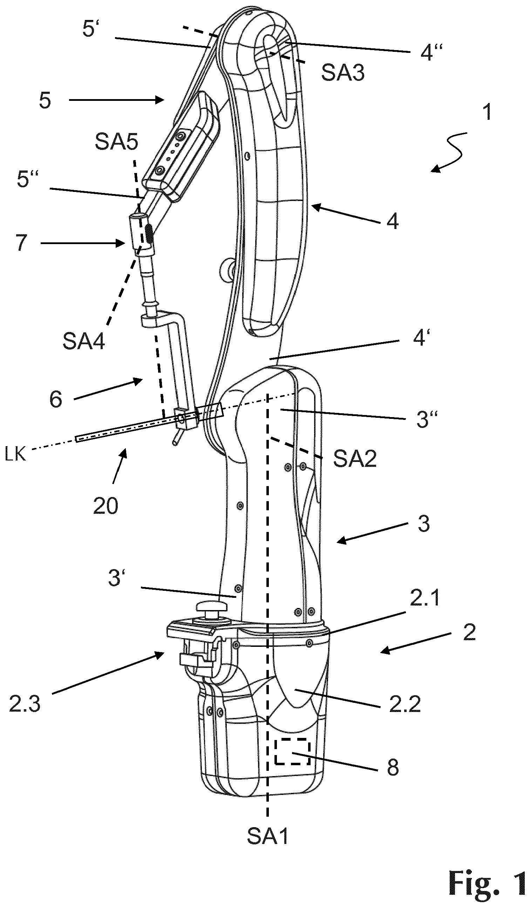

In Figur 1 ist beispielhaft ein Operations-Assistenz-System 1 zur Führung von medizinischen Hilfsinstrumenten 20 bei medizinischen Eingriffen oder Operationen in oder an einem Patientenkörper 10 dargestellt. Unter einem medizinischen Hilfsinstrument 20 im Sinne der Erfindung wird insbesondere eine Endoskopkamera verstanden, die im Wesentlichen aus einem Endoskop 21 und einer an dessen freien Ende angeordnete Kameraeinheit 22 besteht.

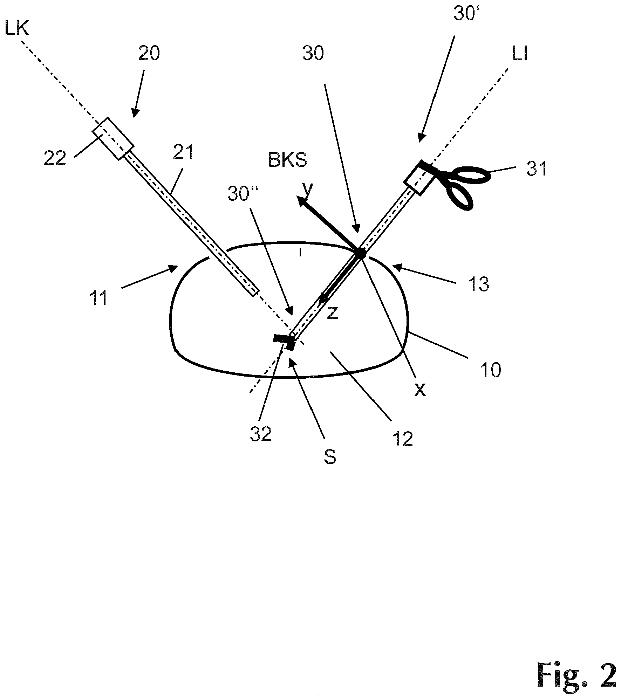

Derartige Endoskopkameras 20 werden häufig bei minimal-invasiven Operationen eingesetzt, und zwar werden diese über eine kleinformatige erste Operationsöffnung 11 in einen Operationsraum 12 innerhalb eines Patientenkörpers 10 eingeführt. Das eigentliche medizinische Instrument bzw. Operationsinstrument 30 zur Durchführung des medizinischen Eingriffs wird über eine zweite Operationsöffnung 13 in den Operationsraum 12 des Patientenkörpers 10 eingeführt. Die erste und zweite Operationsöffnung 11, 13 werden in der Literatur häufig auch als "Trokar" bzw. "Trokarpunkte" bezeichnet. In Figur 2 ist eine typische Operationssituation beispielhaft anhand eines Schnitts durch einen Patientenkörper 10 im minimal-invasiven Operationsbereich schematisch angedeutet.

Mittels der Endoskopkamera 20 bzw. dessen Kameraeinheit 22 werden Bildaufnahmen bzw. Bilder des Operationsraumes 12, u.a. der im Operationsraum 12 befindlichen Spitze S des medizinischen Instrumentes 30 erzeugt, welche dem Operateur während des medizinischen Eingriffs bzw. der minimal-invasiven Operation mittels einer Monitoreinheit angezeigt werden. Der Operateur kann mittels der auf der Monitoreinheit dargestellten aktuellen Bilder ("Livebilder") aus dem Operationsraum 12 den Verlauf der Operation überwachen und das medizinische Instrument bzw. Operationsinstrument 30 entsprechend führen. Um stets ein aktuelles und optimales Bild des Operationsraumes 12, insbesondere des medizinischen Instrumentes bzw.

Operationsinstrumentes 30 vorliegen zu haben, ist für den Operateur eine optimale Ansteuerung bzw. Bedienung, insbesondere auch Ausrichtung und/oder Einstellung der Endoskopkamera 22 von wesentlicher Bedeutung.

Das erfindungsgemäße Operations-Assistenz-System 1 ermöglicht dem Operateur eine hochgenaue und bedienerfreundliche Bedienung der Führung des Hilfsinstruments, insbesondere der Endoskopkamera 20 mittels einer "Sprachsteuerung". Hierzu besteht das Operations-Assistenz-System 1 beispielsweise aus einer Basiseinheit 2 und einer ein System aus mehreren Armen, insbesondere Roboterarmen umfassenden Roboterkinematik, wobei die Roboterkinematik im vorliegenden Ausführungsbeispiel eine Tragsäule 3, einen ersten und zweiten Roboterarm 4, 5 und einen Hilfsinstrumententräger 6 aufweist. Der Hilfsinstrumententräger 6 ist beispielsweise am zweiten Roboterarm 5 angelenkt, und zwar mittels eines abgewinkelten Gelenkstückes 7. Der Hilfsinstrumententräger 6 ist beispielsweise zur direkten Aufnahme des medizinischen Hilfsinstrumentes, und zwar der Endoskopkamera 20 oder zur indirekten Aufnahme mittels einer weiteren, nicht in den Figuren dargestellten Hilfsinstrumentenhalterung ausgebildet. Der beschriebene Aufbau der Roboterkinematik des Operations-Assistenz-Systems 1 ist aus der perspektivischen Darstellung des Operations-Assistenz-Systems 1 gemäß Figur 1 beispielhaft ersichtlich.

Die Basiseinheit 2 umfasst ferner beispielsweise eine Trägerplatte 2.1, ein vorzugsweise mehrteiliges Basisgehäuse 2.2 und zumindest ein Befestigungselement 2.3, mittels dem das vorzugsweise portabel ausgebildete Operations-Assistenz-System 1 bzw. die Basiseinheit 2 beispielsweise seitlich an einem Operationstisch (nicht in den Figuren dargestellt) befestigbar ist. Im Basisgehäuse 2.2 sind zumindest eine Steuereinrichtung 8 und ggf. weitere Funktionseinheiten aufgenommen, die ggf. mit einem nicht dargestellten Computersystem zusammenwirken.

Die Tragsäule 3 weist einen unteren und oberen Endabschnitt 3', 3" auf. Die Basiseinheit 2 des Operations-Assistenz-Systems 1 ist mit dem unteren Endabschnitt 3' der Tragsäule 3 der Roboterkinematik gesteuert schwenkbar um eine erste Schwenkachse SA1 verbunden. Die erste Schwenkachse SA1 verläuft hierbei vertikal zur Aufstellungsebene des Operations-Assistenz-Systems 1 bzw. zur Operationsebene bzw. Ebene eines Operationstisches.

Ferner weist der erste Roboterarm 4 einen ersten und zweiten Endabschnitt 4', 4" auf, wobei der erste Endabschnitt 4' des ersten Roboterarmes 4 mit dem der Basiseinheit 2 gegenüberliegenden oberen Endabschnitt 3" der Tragsäule 3 gesteuert schwenkbar um eine zweite Schwenkachse SA2 und der zweite Endabschnitt 4" des ersten Roboterarmes 4 mit einem ersten Endabschnitt 5' des zweiten Roboterarmes 5 gesteuert schwenkbar um eine dritte Schwenkachse SA3 verbunden ist.

Der zweite Roboterarm 5 weist einen zweiten, dem ersten Endabschnitt 5' gegenüberliegenden Endabschnitt 5" auf, an dem im vorliegenden Ausführungsbeispiel das abgewinkelte Gelenkstücks 7 drehbar um eine vierte Schwenkachse SA4 vorgesehen ist. Das abgewinkelte Gelenkstücks 7 ist zur drehbaren und lösbaren Aufnahme eines Anschlussabschnittes des Hilfsinstrumententrägers 6 ausgebildet, und zwar um eine fünfte Schwenkachse SA5. Das gegenüberliegende freie Ende des Hilfsinstrumententrägers 6 bildet eine Instrumentenaufnahme aus.

Die erste Schwenkachse SA1 verläuft senkrecht zur Aufstellungsebene bzw. Operationsebene und die zweite und dritte Schwenkachse SA2, SA3 verlaufen parallel zueinander, wohingegen die erste Schwenkachse SA1 senkrecht zur zweiten oder dritten Schwenkachse SA2, SA3 orientiert ist.

Zum Antrieb der Roboterkinematik des Operations-Assistenz-Systems 1 sind mehrere, nicht in den Figuren dargestellte Antriebseinheiten vorgesehen, die über zumindest eine Steuereinrichtung 8 vorzugsweise unabhängig voneinander ansteuerbar ausgebildet sind. Die Antriebseinheiten sind vorzugsweise in der Basiseinheit 2, der Tragsäule 3 und/oder in den Roboterarmen 4 bis 6 integriert bzw. darin aufgenommen. Beispielsweise können die Antriebseinheiten durch hydraulische Antriebe oder elektrische Antriebe, insbesondere Linearmotoreinheiten oder Spindelmotoreinheiten gebildet sein.

Die zumindest eine Steuereinrichtung 8 ist vorzugsweise in der Basiseinheit 2 des Operations-Assistenz-Systems 1 aufgenommen und dient zur Erzeugung von Steuersignalen zur Ansteuerung der Antriebe bzw. Antriebseinheiten zum gesteuerten motorisches Schwenken der Roboterkinematik um die vorgegebenen Schwenkachsen SA1 bis SA5 und/oder Halten der Roboterkinematik in einer vorgegebenen Halteposition in einem kartesischen Koordinatensystem.

Die Tragsäule 3 erstreckt sich vertikal, und zwar im Wesentlichen entlang der ersten Schwenkachse SA1, d.h. ist näherungsweise um Ihre eigene Längsachse drehbar ausgebildet. Der erste und zweite Roboterarm 4, 5 erstrecken sich im Wesentlichen ebenfalls entlang einer Geraden, die vorzugsweise senkrecht zur zweiten bzw. dritten Schwenkachse SA2, SA3 verläuft. Im vorliegenden Ausführungsbeispiel weist zumindest der erste Roboterarm 4 eine leichte Krümmung auf.

Zur Einstellung der Ausgangsposition des Operations-Assistenz-Systems 1 bzw. zur Kalibrierung der Steuereinrichtung 8 in Bezug auf die erste Operationsöffnung 11 bzw. den Trokarpunkt, durch die bzw. den das medizinische Hilfsinstrument 20 in den Operationsraum eingeführt wird, ist eine Registrierungsroutine vorgesehen, mittels der vor der Operation das Operations-Assistenz-System 1 beispielhaft dadurch registriert wird, dass ein in den Figuren nicht gezeigter Registriertaster an denjenigen Bereich des auf dem OP-Tisch bereits platzierten Patienten geführt wird, an dem die erste Operationsöffnung 11 zum Einführen des medizinischen Hilfsinstruments 20 vorgesehen ist. Nach dieser Kalibrierung ist das Operations-Assistenz-System 1 zur Führung des medizinischen Hilfsinstrumentes, insbesondere Endoskopkamera 20 einsatzbereit.

In Figur 2 ist beispielhaft eine schematische Seitenansicht einer in den Operationsraum 12 des Patientenkörpers 10 über die erste Operationsöffnung 11 eingeführten Endoskopkamera 20 dargestellt. Ferner zeigt Figur 2 ein über die zweite Operationsöffnung 13 in den Operationsraum 12 eingeführtes medizinisches Instrument bzw. Operationsinstrument 30. Die zweite Operationsöffnung 12 bildet hierbei beispielsweise den Ursprung eines Bezugskoordinatensystems BKS mit dem Raumachsen x, y, z.

In Figur 2 erstreckt sich beispielhaft die x-Achse des kartesischen Bezugskoordinatensystems BKS senkrecht zur Zeichenebene, die y-Achse des kartesischen Bezugskoordinatensystems BKS senkrecht zur Längsachse LI des medizinischen Instruments 30, wohingegen die z-Achse entlang der Längsachse LI des medizinischen Instruments 30 verläuft bzw. mit dieser zusammenfällt.

Der Ursprung kommt im Bereich der zweiten Operationsöffnung 12 zu liegen. Vorteilhaft ist bei einer derartigen Orientierung des kartesischen Bezugskoordinatensystems BKS eine Drehung um die Längsachse LI des medizinischen Instruments 30 jeweils eine Drehung um die z-Achse, welche eine vereinfachte Auswertung einer Drehbewegung um die Längsachse LI des medizinischen Instruments 30 ermöglicht.

Das medizinische Instrument bzw. Operationsinstrument 30 weist beispielsweise an einem freien Ende 30' zumindest ein, vorzugsweise zwei Griffelemente 31 auf, die beispielsweise in Form zweier Griffringe mit jeweils anschließenden Verbindungsschaft ausgebildet sind. Über zumindest eines der Griffelemente 31 ist ein am gegenüberliegenden freien Ende 30" des medizinischen Instruments bzw. Operationsinstrumentes 30 angeordnetes Funktionselement 32, beispielsweise ein Greif- oder Schneidelement betätigbar. Das Funktionselement 32 bildet hierbei die Spitze S des medizinischen Instrumentes 30 aus, welches sich während der Operation im Operationsraum 12 befindet und von der Endoskopkamera 20 erfasst wird. Das freie Ende 30' befindet sich außerhalb des Patientenkörpers 10 und bildet den Griffbereich des medizinisches Instrument bzw. Operationsinstrument 30 aus.

Zur Steuerung der Bewegung des Hilfsinstruments 20 im Operationsraum 12 ist eine Steuereinheit CU vorgesehen, mittels der vorzugsweise auf das Bezugskoordinatensystems BKS bezogene Steuersignale SS erzeugt werden. Diese werden an die Steuereinrichtung 8 übertragen, mittels der eine entsprechende Ansteuerung der Antriebe bzw. Antriebseinheiten der Roboterkinematik zum gesteuerten motorisches Schwenken der Tragsäule 3 und/oder der Roboterarme 4, 5 der Roboterkinematik erfolgt, um eine Dreh- und/oder Schwenkbewegung um die vorgegebenen Schwenkachsen SA1 bis SA5 und/oder ein Halten der Roboterkinematik in einer vorgegebenen Halteposition bezogen auf das Bezugskoordinatensystems BKS zu bewirken.

Erfindungsgemäß weist die Steuereinheit CU zumindest eine Sprachsteuerroutine SSR auf, welche zur Erzeugung der Steuersignale SS über Sprachbefehle bzw. Sprachkommandos SB, SB1, SB2 des Operateurs während der Operation ermöglicht.

Eine derartige "Sprachsteuerung" der Roboterkinematik und damit der Bewegung des Hilfsinstruments 20 im Operationsraum 12 ermöglicht dem Operateur durch entsprechende vorgegebene Sprachbefehle SB, SB1, SB2 das Hilfsinstrument, insbesondere eine Endoskopkamera 20 dem medizinischen Instrument 30 nachzuführen und/oder das von Kameraeinheit 22 erzeugte Abbildung des Operationsraumes 12 entsprechend seinen aktuellen Bedürfnissen durch entsprechende Bewegung der Endoskopkamera 20 zu verändern. Der Einsatz von Sprachsteuerungen zur zumindest teilweisen Steuerung von Operationsrobotern ist bekannt, wobei bekannte System nur einen eingeschränkten Funktionsumfang und eine relativ geringe Steuergenauigkeit aufweisen.

Hier setzt die Erfindung an und ermöglicht dem Operateur eine genauere und auch benutzerfreundliche Sprachsteuerung. Erfindungsgemäß ist die Sprachsteuerroutine SSR zur Erfassung und Auswertung von aus zumindest einem ersten und zweiten Sprachbefehl SB1, SB2 bestehenden Sprachbefehlskombinationen SBK ausgebildet, wobei dem ersten Sprachbefehl SB1 zumindest eine Richtungsinformation RI oder eine Geschwindigkeitsinformation VI und dem zweiten Sprachbefehl SB2 zumindest eine Betragsinformation BI jeweils bezogen auf das kartesische Bezugskoordinatensystem BKS zugeordnet sind. Es versteht sich, dass eine Sprachbefehlskombination SBK selbstverständlich auch mehr als zwei Sprachbefehle SB1, SB2 etc. umfassen kann, so dass beispielsweise neben einer Richtungs- oder Geschwindigkeitsinformation und der Betragsinformation RI, BI auch noch weitere Bewegungsdaten in Form von weiteren Sprachbefehlen SB an die Sprachsteuerroutine SSR übergeben werden können. Im vorliegenden Ausführungsbeispiel wird jedoch die Erfindung anhand einer zwei Sprachbefehle SB1, SB2 umfassenden Sprachbefehlskombinationen SBK näher erläutert. Die Erfindung ist jedoch keinesfalls darauf beschränkt.

Die Steuereinheit CU weist zumindest eine Prozessoreinheit CPU sowie eine mit dieser verbundene Sprachsignalerfassungseinheit SEU und beispielweise eine Speichereinheit MU auf. Die Sprachsignalerfassungseinheit SEU ist zur Erfassung des vom Operateur gesprochenen Steuerbefehls SB, SB1, SB2 in Form von beispielsweise ein oder mehreren Wörtern umfassenden Sprachsignalen ausgebildet. Das entsprechend über die Sprachsignalerfassungseinheit SEU erfasste Sprachsignal bildet den Steuerbefehl SB, SB1, SB2, der an die in der Prozessoreinheit CPU ausgeführte Sprachsteuerroutine SSR übertragen wird. In der Speichereinheit MU der Steuereinheit CU kann jeder erfindungsgemäßen Sprachbefehlskombinationen SBK, die aus den zur Verfügung stehenden Steuerbefehlen SB1, SB2 erzeugbar sind, ein Steuersignal SS zugeordnet sein, welches eine entsprechende Ansteuerung der Roboterkinematik durch die Steuereinrichtung 8 bewirkt. Alternativ kann ausgehend von den erfassten und ausgewerteten Steuerbefehlen SB1, SB2 auch ein geeignetes Steuersignal SS ermittelt werden.

Zusätzlich zur erfindungsgemäßen Sprachsteuerung kann auch eine manuell betätigbare Eingabeeinheit, beispielsweise in Form einer Fernbedienung, eines Joysticks oder einer sonstigen mehrere Schaltelemente aufweisenden Eingabeeinheit vorgesehen sein, über welche alternativ oder zusätzlich zur Sprachsteuerroutine SSR die erforderlichen Steuersignale SS erzeugbar sind. Der Operateur kann dann zwischen unterschiedlichen Eingabeeinheiten wählen.

Erfindungsgemäß werden von der Sprachsteuerroutine SSR die vom Operateur erzeugten Sprachbefehle, und zwar der erste und zweite Sprachbefehle SB1, SB2, die zeitlich unmittelbar aufeinander folgen, innerhalb einer vorgegebenen Zeitdauer erfasst und ausgewertet. Abhängig von den über die Sprachbefehle SB1, SB2 erfassten Richtungs- oder Geschwindigkeitsinformation RI, VI und der weiteren Betragsinformationen BI einer Sprachbefehlskombination SBK wird zumindest ein Steuersignal SS erzeugt bzw. ermittelt, über welches zumindest die Bewegung des medizinischen Hilfsinstrumentes 20 richtungs- und betragsmäßig gesteuert wird.

Ein erster oder zweiter Sprachbefehl SB1, SB2 im erfindungsgemäßen Sinne kann ein oder mehrere Wörter oder Wortbestandteile umfassen, die entweder alleine oder in Kombination unmittelbar nacheinander ausgesprochen werden und denen jeweils eine Richtungsinformation RI bzw. Geschwindigkeitsinformation VI bzw. Betragsinformationen BI in der Sprachsteuerroutine SSR zugeordnet ist bzw. sind.

Zur Ansteuerung stehen hierbei dem Operateur erste Sprachbefehle SB1 mit einer vorgegebenen Anzahl an unterschiedlichen Richtungsinformationen RI oder unterschiedlichen Geschwindigkeitsinformationen und zweite Sprachbefehle SB2 mit einer vorgegebenen Anzahl an unterschiedlichen Betragsinformationen BI zur Verfügung. Ausgegangen wird hierbei jeweils von der aktuell vorliegenden Istposition des Hilfsinstruments 20 im Bezugskoordinatensystem BKS zum Erreichen einer über die Sprachbefehlskombination SBK vorgegebenen Sollposition im Bezugskoordinatensystem BKS.

Unter Richtungsinformation RI im erfindungsgemäßen Sinne wird eine definierte Bewegungsrichtung des Hilfsinstruments 20 im Bezugskoordinatensystems BKS von einer Ist- zu einer Sollposition verstanden, welche vorzugsweise in Form eines Richtungsvektors vorgegeben ist, der von der Istposition zur Sollposition orientiert ist.

Unter der Betragsinformation BI wird im erfindungsgemäßen Sinne der Betrag der Bewegung des Hilfsinstrumentes 20 entlang der von Richtungsinformation RI bzw. vom Richtungsvektor vorgegebenen Bewegungsrichtung im Bezugskoordinatensystems BKS verstanden, wobei der Betrag im Wesentlichen die bei der Bewegung zurückzulegende Weglänge bzw. Wegstrecke ausgehend von der Istposition des Hilfsinstruments 20 zum Erreichen der gewünschten Sollposition angibt. Über die erfindungsgemäße Kombination sowohl von Richtungsinformationen RI als auch Betragsinformationen BI in einer Sprachbefehlskombination SBK ist eine noch genauere Ansteuerung einer gewünschten Sollposition im Bezugskoordinatensystems BKS möglich, die zudem äußerst benutzerfreundlich für den Operateur ist.

Unter Geschwindigkeitsinformation VI im erfindungsgemäßen Sinne wird eine oder mehrere vorgegebene Geschwindigkeiten verstanden, die die die Bewegungsgeschwindigkeit angegeben, mit welcher das Hilfsinstrumentes 20 über die Roboterkinematik im kartesischen Bezugskoordinatensystem BKS bewegt wird.

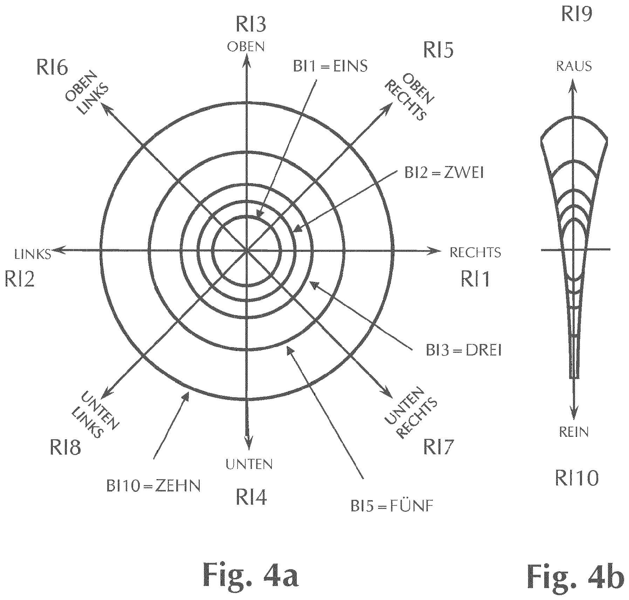

In den Figuren 4a und 4b sind beispielhaft in einem schematischen Diagramm unterschiedliche Richtungsinformationen RI1 - RI10 und Betragsinformationen BI1 - BI10 dargestellt, die Gegenstand eines ersten bzw. zweiten Sprachbefehls SB1, SB2 sein können.

In Figur 4a ist beispielsweise eine Draufsicht auf die x-y-Ebene des Bezugskoordinatensystems BKS und in Figur 4b eine Draufsicht auf die x-z-Ebene des Bezugskoordinatensystems BKS dargestellt und darin die unterschiedlichen Richtungs- und Betragsinformationen RI, BI beispielhaft eingezeichnet.

Figur 4a zeigt beispielsweise einen ersten Sprachbefehl SB1 mit dem Wortlaut "RECHTS", dem eine erste Richtungsinformation RI1 zugeordnet ist, welche mit der x-Achse zusammenfällt und in positiver x-Richtung weist. Ferner ist ein erster Sprachbefehl SB1 mit dem Wortlaut "LINKS" vorgesehen, dem eine zweite Richtungsinformation RI2 zugeordnet ist. Die zweite Richtungsinformation RI2 entspricht wiederum der x-Achsenrichtung, jedoch in negativer x-Richtung. Analog dazu sind in erster Sprachbefehl SB1 mit dem Wortlaut "OBEN" mit einer dritten Richtungsinformation RI3 und ein alternativer erster Sprachbefehl SB1 mit dem Wortlauft "UNTEN" mit einer vierten Richtungsinformation RI4 vorgesehen. Die dritte und vierte Richtungsinformation RI3 entsprechen der y-Achsenrichtung, und zwar zum einen in positiver und zum anderen in negativer y-Richtung. Schließlich stehen beispielhaft noch als erste Sprachbefehle SB1 der Wortlaut "OBEN RECHTS" betreffend eine fünfte Richtungsinformation RI5, der Wortlaut "OBEN LINKS" betreffend eine sechste Richtungsinformation RI6, der Wortlaut "UNTEN RECHTS betreffend eine siebte Richtungsinformation RI7 sowie der Wortlaut "UNTEN LINKS" betreffend eine achte Richtungsinformation RI8 in der x-y-Ebene zur Verfügung. Die fünfte Richtungsinformation RI5 weist ausgehend vom Zentrum des Bezugskoordinatensystem BKS als Winkelhalbierende der jeweils positiven x- und y- Achse in positiver x- und y-Richtung. Die sechste Richtungsinformation RI6 weist ausgehend vom Zentrum des Bezugskoordinatensystem BKS als Winkelhalbierende der negativen x-Achse und der positiven y-Achse in negativer x-Richtung und in positiver y- Richtung. Die siebte Richtungsinformation RI7 weist ausgehend vom Zentrum des Bezugskoordinatensystem BKS als Winkelhalbierende der positiven x-Achse und der negativen y-Achse in positiver x-Richtung und in negativer y- Richtung. Die achte Richtungsinformation RI8 weist ausgehend vom Zentrum des Bezugskoordinatensystem BKS als Winkelhalbierende der jeweils negativen x-und y- Achse in negativer x- und y- Richtung. Die fünfte bis achte Richtungsinformationen RI5 bis RI8 geben damit in der x-y-Eben des Bezugskoordinatensystems BKS diagonal verlaufende Bewegungsrichtungen an.

Schließlich sind in Figur 4b beispielhaft noch zwei weitere Optionen eines ersten Sprachbefehls SB1 dargestellt, und zwar eine dem Wortlaut "RAUS" zugeordnete neunte Richtungsinformation RI9, die ausgehend vom Zentrum des Bezugskoordinatensystem BKS mit der z-Achse zusammenfällt und sich in negativer z-Richtung erstreckt sowie eine dem Wortlaut "REIN" zugeordnete zehnte Richtungsinformation RI10, die ausgehend vom Zentrum des Bezugskoordinatensystem BKS ebenfalls mit der z-Achse zusammenfällt, sich jedoch in positiver z-Richtung erstreckt. Damit stehen im vorliegenden Ausführungsbeispiel 10 unterschiedliche Bewegungsinformationen RI1 bis RI10 dem Operateur für die Steuerung der Bewegung des Hilfsinstrumentes 20 bzw. der Endoskopkamera zur Verfügung, die beispielsweise jeweils ein oder zwei Wörter umfassen.

Erfindungsgemäß umfasst eine Sprachbefehlskombination SBK hierzu zusätzlich noch eine individuell zur jeweiligen Richtungsinformation RI1 bis RI10 kombinierbare Betragsinformationen BI1 bis BI10, welche den zweiten Sprachbefehl SB2 bildet und die sich vorzugsweise an den Wortlaut des ersten Sprachbefehles SB2 anschließt. Im vorliegenden Ausführungsbeispiel sind eine erste bis zehnte Betragsinformation BI1 bis BI10 vorgesehen, welches jeweils einen Betrag bzw. Weglänge entlang der durch die jeweilige Richtungsinformation RI1 bis RI10 vorgegebene Bewegungsrichtung angeben.

Einer Betragsinformation BI1 bis BI10 kann beispielsweise ein Zeitintervall zugeordnet sein, welches die Zeitdauer der Bewegung des Hilfsinstrumentes entlang der vorgegebenen Bewegungsrichtung angibt. Ausgehend von einer ebenfalls über zugeordnete Sprachbefehle SB einstellbare, vorzugsweise konstanten Bewegungsgeschwindigkeit ergibt sich dann eine in diesen Zeitintervall zurückgelegte Wegstrecke bzw. Weglänge. In einer bevorzugten, im vorliegenden Ausführungsbeispiel dargestellten Ausführungsvariante betreffend die erste bis zehnte Betragsinformation BI1 bis BI10 jeweils ein Vielfaches einer vorgegebenen Referenzweglänge bzw. Referenzwegstrecke, d.h. die erste Betragsinformation BI1 entspricht dem einfachen der Referenzweglänge, die zweite Betragsinformation BI2 dem zweifachen usw.. Entsprechend kann der zweite Sprachbefehl SB2 einen unterschiedlichen Wortlaut aufweisen. Im vorliegenden Ausführungsbeispiel ist beispielsweise dem Wortlaut "EINS" die erste Betragsinformation BI1, dem Wortlaut "ZWEI" die zweite Betragsinformation BI2, dem Wortlaut "DREI" die dritte Betragsinformation BI3 usw. zugeordnet. In Fig. 4a sind beispielshaft nur die erste bis dritte, fünfte und zehnte Betragsinformation BI1 bis BI3, BI5 und BI10 dargestellt. Der vom Operateur auszusprechende Wortlaut des zweiten Sprachbefehls SB2 entspricht damit der jeweiligen Zahl von 1 bis 10 in englischer Sprache. Die zugeordnete Zahl gibt jeweils das Vielfache der der ersten Betragsinformation BI1 zugeordneten Referenzweglänge an.

In einer alternativen, nicht in den Figuren explizit dargestellten Ausführungsvariante können selbstverständlichen Betragsinformationen BI1 bis BI10 vorgesehen sein, welche kein Vielfaches einer Referenzweglänge darstellen, sondern individuell festgelegt sind. Vorzugsweise sind mindestens zwei Betragsinformation BI1 bis BI10 mit unterschiedlichen Beträgen bzw. Weglängen bzw. Geschwindigkeiten mit den vorgenannten Richtungsinformationen RI1 bis RI10 kombinierbar.

Auch kann anstelle einer Zeitintervalls auch einer Betragsinformation BI1 bis BI10 eine konkrete Weglänge bzw. Wegstrecke zugeordnet sein, welcher individuell gewählt oder vom Operateur zur Personalisierung seines Systems einstellbar ist.

In einer alternativen, nicht in den Figuren dargestellten Ausführungsvariante kann zumindest eine Geschwindigkeitsinformation VI vorgesehen sein, der als erster Sprachbefehl SB1 der Wortlaut "GESCHWINDIGKEIT" zugeordnet ist und der eine vorgegebene Referenzgeschwindigkeit zugeordnet ist. Der zweite Sprachbefehl SB2 der Sprachbefehlskombination SBK kann analog zum zuvor beschriebenen Ausführungsbeispiel unterschiedliche Betragsinformation BI1 bis BI10 aufweisen, die jeweils ein Vielfaches der Referenzgeschwindigkeit vorgeben.

In einer bevorzugten Ausführungsvariante wird die Sprachsteuerung über die Sprachsteuerroutine SSR ausschließlich bei Betätigung eines Aktivierungselementes, vorzugsweise eines Aktivierungsschalters oder -tasters durch den Operateur oder durch einen vorgegebenen Aktivierungssprachbefehl vom Operateur aktiviert.

Auch kann die aktuelle Istposition des Hilfsinstrumentes 20 im kartesischen Bezugskoordinatensystem über einen Sprachsteuerbefehl SB in der Speichereinheit MU der Steuereinheit CU gespeichert werden. Durch Eingabe eines weiteren Sprachbefehls SB kann die gespeicherte Istposition wieder gezielt zu einem späteren Zeitpunkt angefahren werden.

### Bezugszeichen liste

- 1: Operations-Assistenz-System
- 2: Basiseinheit
- 2.1: Trägerplatte
- 2.2: Basisgehäuse
- 2.3: Befestigungselement
- 3: Tragsäule
- 3': unteren Endabschnitt
- 3": oberer Endabschnitt
- 4: erster Roboterarm
- 4': erster Endabschnitt
- 4": zweiter Endabschnitt
- 5: zweiter Roboterarm
- 5': erster Endabschnitt
- 5": zweiter Endabschnitt
- 6: Hilfsinstrumententräger
- 7: abgewinkeltes Gelenkstück
- 8: Steuereinrichtung

- 10: Patientenkörper
- 11: erste Operationsöffnung ("Trokar")
- 12: Operationsraum
- 13: zweite Operationsöffnung ("Trokar")

- 20: Hilfsinstrument, insbesondere Endoskopkamera
- 21: Endoskop
- 22: Kameraeinheit

- 30: medizinisches Instrument
- 30': freies Ende bzw. Griffbereich
- 30": freies Ende
- 31: Griffelemente
- 32: Funktionselement

- BI: Betragsinformation
- BI1 bis BI10: erste bis zehnte Betragsinformationen
- BKS: kartesisches Bezugskoordinatensystem
- CU: Steuereinheit
- LI: Längsachse
- LK: Längsachse
- RI: Richtungsinformation
- RI1 bis RI10: erste bis zehnte Richtungsinformationen
- S: Spitze des medizinischen Instrumentes
- SA1: erste Schwenkachse
- SA2: zweite Schwenkachse
- SA3: dritte Schwenkachse
- SA4: vierte Schwenkachse
- SA5: fünfte Schwenkachse
- SB: Steuerbefehl
- SB1: erster Steuerbefehl
- SB2: zweiter Steuerbefehl
- SBK: Steuerbefehlskombination
- SS: Steuersignale
- VI: Geschwindigkeitsinformation

- x, y, z: Raumachsen des kartesischen Bezugskoordinatensystem (BKS)

## Patentansprüche

1. Operations-Assistenz-System zur Führung eines medizinischen Hilfsinstrumentes (20), insbesondere einer Endoskopkamera, welches über eine erste Operationsöffnung (11) in einem Operationsraum (12) eines Patientenkörpers (10) zumindest abschnittsweise einführbar und gesteuert bewegbar ist, umfassend eine das medizinische Hilfsinstrument (20) mittels einer Hilfsinstrumentenhalterung (6) freiendseitig aufnehmende Roboterkinematik (3, 4, 5), die zur Führung des medizinischen Hilfsinstruments (20) im Operationsraum (12) motorisch gesteuert bewegbar ist, und zwar über von einer Steuereinheit (CU) erzeugten Steuersignalen (SS), wobei in der Steuereinheit (CU) zumindest eine Sprachsteuerroutine (SSR) ausgeführt wird, über die unterschiedliche Sprachbefehle (SB, SB1, SB2) erfasst, ausgewertet und abhängig davon zugeordnete Steuersignale (SS) ermittelt werden, **dadurch gekennzeichnet, dass** die Sprachsteuerroutine (SSR) zur Erfassung und Auswertung von aus zumindest einem ersten und zweiten Sprachbefehl (SB1, SB2) bestehenden Sprachbefehlskombinationen (SBK) ausgebildet ist, wobei dem ersten Sprachbefehl (SB1) eine Richtungs- oder Geschwindigkeitsinformation (RI, VI, RI1 bis RI10) und dem zweiten Sprachbefehl (SB2) eine Betragsinformation (BI, BI1 bis BI10) in einem Bezugskoordinatensystem (BKS) zugeordnet sind, und dass abhängig von der erfassten Richtungs- oder Geschwindigkeitsinformation (RI, VI, RI1 bis RI10) und der erfassten Betragsinformation Betragsinformation (BI, BI1 bis BI10) der Sprachbefehlskombination (SBK) zumindest ein zugeordnetes Steuersignal (SS) ermittelt wird, über welches zumindest die Bewegung des medizinischen Hilfsinstrumentes (20) richtungs- oder geschwindigkeitsmäßig und betragsmäßig gesteuert wird, wobei die Richtungsinformation (RI, RI1 bis RI10) die Bewegungsrichtung des Hilfsinstrumentes in dem kartesischen Bezugskoordinatensystem (BKS) und die Betragsinformation (BI, BI1 bis BI10) die Weglänge bzw. Wegstrecke entlang der Bewegungsrichtung in dem kartesischen Bezugskoordinatensystem (BKS) angibt, wobei die Sprachsteuerroutine (SSR) zur Erfassung und Auswertung von zeitlich unmittelbar aufeinander folgenden ersten und zweiten Sprachbefehlen (SB1, SB2) innerhalb einer vorgegebenen Zeitdauer eingerichtet ist.

2. Operations-Assistenz-System nach Anspruch 1, **dadurch gekennzeichnet, dass** der erste und zweite Sprachbefehl (SB1, SB2) ein oder mehrere Wörter umfasst.

3. Operations-Assistenz-System nach Anspruch 1 oder 2, **dadurch gekennzeichnet, dass** die Sprachsteuerung mittels der Sprachsteuerroutine (SSR) ausschließlich bei Betätigung eines Aktivierungselementes, vorzugsweise eines Aktivierungsschalters oder - tasters durch den Operateur oder bei Vorliegen eines Aktivierungssprachbefehls aktiviert wird.

4. Operations-Assistenz-System nach einem der vorhergehenden Ansprüche, **dadurch gekennzeichnet, dass** mittels eines dritten Sprachbefehls die Istposition des Hilfsinstrumentes (20) bezogen auf das Bezugskoordinatensystem (BKS) speicherbar und mittels eines vierten Sprachbefehls wieder gezielt anfahrbar ist.

5. Verfahren zur Erzeugung von Steuersignalen (SS) zur Ansteuerung einer motorisch gesteuert bewegbaren Roboterkinematik (3, 4, 5) eines Operations-Assistenz-Systems zur Führung eines medizinischen Hilfsinstrumentes (20), insbesondere einer Endoskopkamera, bei dem das medizinische Hilfsinstrument (20) mittels einer Hilfsinstrumentenhalterung (6) freiendseitig an der Roboterkinematik (3, 4, 5) angeordnet ist, bei dem das medizinische Hilfsinstrumentes (20) über eine erste Operationsöffnung (11) zumindest abschnittsweise in den Operationsraum (12) eines Patientenkörpers (10) einführbar ist und bei dem die Steuersignale (SS) mittels zumindest einer, in einer Steuereinheit (CU) ausgeführten Sprachsteuerroutine (SSR) ermittelt werden, **dadurch gekennzeichnet, dass** mittels der Sprachsteuerroutine (SSR) eine aus zumindest einem ersten und zweiten Sprachbefehl (SB1, SB2) bestehende Sprachbefehlskombination (SBK) erfasst und ausgewertet wird, wobei dem ersten Sprachbefehl (SB1) eine Richtungs- oder Geschwindigkeitsinformation (RI, VI, RI1 bis RI10) und dem zweiten Sprachbefehl (SB2) eine Betragsinformation (BI, BI1 bis BI10) in einem Bezugskoordinatensystem (BKS) zugeordnet sind, und dass abhängig von der erfassten Richtungs- oder Geschwindigkeitsinformation (RI, VI, RI1 bis RI10) und der erfassten Betragsinformation (BI, BI1 bis BI10) der Sprachbefehlskombination (SBK) zumindest ein zugeordnetes Steuersignal (SS) ermittelt wird, über welches zumindest die Bewegung des medizinischen Hilfsinstrumentes (20) richtungs- oder geschwindigkeitsmäßig und betragsmäßig gesteuert wird, wobei die Richtungsinformation (RI, RI1 bis RI10) die Bewegungsrichtung des Hilfsinstrumentes in dem kartesischen Bezugskoordinatensystem (BKS) und die Betragsinformation (BI, BI1 bis BI10) die Weglänge bzw. Wegstrecke entlang der Bewegungsrichtung in dem kartesischen Bezugskoordinatensystem (BKS) angibt, wobei zeitlich unmittelbar aufeinander folgende erste und zweite Sprachbefehle (SB1, Sb2) von der Sprachsteuerroutine (SSR) innerhalb einer vorgegebenen Zeitdauer erfasst und ausgewertet werden.

## Claims

1. Surgery assistance system for guiding a medical auxiliary instrument (20), in particular an endoscope camera, at least a section of which can be introduced through a first surgical opening (11) and moved in controlled manner in an operating space (12) in a patient's body (10), comprising a robot kinematics system (3, 4, 5), the distal end of which accommodates the medical auxiliary instrument (20) by means of an auxiliary instrument carrier (6), which is movable for guiding the medical auxiliary instrument (20) in the operating space (12) in controlled manner by motorised means, via control signals (SS) generated by a control unit (CU), wherein at least one voice control routine (SSR) is executed in the control unit (CU), via which various voice commands (SB, SB1, SB2) are captured, evaluated, and assigned control signals (SS) are calculated on the basis thereof, **characterized in that** the voice control routine (SSR) is designed to capture and evaluate voice command combinations (SBK) consisting of at least a first and a second voice command (SB1, SB2), wherein an item of direction or speed information (RI, VI, RI1 to RI10) is assigned to the first voice command (SB1) and an item of magnitude information (BI, BI1 to BI10) is assigned to the second voice command (SB2) in a reference coordinate system (BKS), and that at least one assigned control signal (SS) is calculated on the basis of the captured direction or speed information (RI, VI, RI1 to RI10) and the captured magnitude information (BI, BI1 to BI10) of the voice command combination (SBK), via which signal at least the movement of the medical auxiliary instrument (20) is controlled in terms of direction or speed and magnitude, wherein the direction information (RI, RI1 to RI10) specifies the direction of movement of the auxiliary instrument in the Cartesian reference coordinate system (BKS) and the magnitude information (BI, BI1 to BI10) specifies the travel distance or travel path along the direction of movement in the Cartesian reference coordinate system (BKS), wherein the voice control routine (SSR) is configured to capture and evaluate temporally immediately consecutive first and second voice commands (SB1, SB2) within a predetermined time period.

2. Surgery assistance system according to Claim 1, **characterized in that** the first and second voice commands (SB1, SB2) comprise one or more words.

3. Surgery assistance system according to Claim 1 or 2, **characterized in that** the voice control is activated by means of the voice control routine (SSR) solely upon actuation of an activation element, preferably an activation switch or button by the surgeon or when an activation voice command exists.

4. Surgery assistance system according to any one of the preceding claims, **characterized in that** the actual position of the auxiliary instrument (20) relative to the reference coordinate system (BKS) can be stored by means of a third voice command, and the position can be regained precisely by means of a fourth voice command.

5. Method for generating control signals (SS) for actuating a robot kinematic system (3, 4, 5) of a surgery assistance system that is movable in controlled manner by motorised means for guiding a medical auxiliary instrument (20), in particular an endoscope camera, in which the medical auxiliary instrument (20) is arranged on the distal end of the robot kinematic system (3, 4, 5) by means of an auxiliary instrument carrier (6), in which at least a section of the medical auxiliary instrument (20) can be introduced into the operating space (12) of a patient's body (10) through a first surgical opening (11), and in which the control signals (SS) are calculated by means of at least one voice control routine (SSR) executed in a control unit (CU), **characterized in that** a voice command combination (SBK) consisting of at least a first and a second voice command (SB1, SB2) is captured and evaluated by means of the voice control routine (SSR), wherein an item of direction or speed information (RI, VI, RI1 to RI10) is assigned to the first voice command (SB1) and an item of magnitude information (BI, BI1 to BI10) is assigned to the second voice command (SB2) in a reference coordinate system (BKS), and that at least one assigned control signal (SS) is calculated on the basis of the captured direction or speed information (RI, VI, RI1 to RI10) and the captured magnitude information (BI, BI1 to BI10) of the voice command combination (SBK), via which signal at least the movement of the medical auxiliary instrument (20) is controlled in terms of direction or speed and magnitude, wherein the direction information (RI, RI1 to RI10) specifies the direction of movement of the auxiliary instrument in the Cartesian reference coordinate system (BKS) and the magnitude information (BI, BI1 to BI10) specifies the travel distance or travel path along the direction of movement in the Cartesian reference coordinate system (BKS), temporally immediately consecutive first and second voice commands (SB1, SB2) are captured and evaluated within a predetermined time period by the voice control routine (SSR).

## Revendications

1. Système d'assistance chirurgical pour le guidage d'un instrument médical auxiliaire (20), en particulier d'une caméra endoscopique qui est introductible et mobile du moins par sections de manière contrôlée par une première ouverture chirurgicale (11) dans un espace d'opération (12) d'un corps de patient (10), comprenant une cinématique robotique (3, 4, 5) recevant à une extrémité libre l'instrument médical auxiliaire (20) au moyen d'un support d'instrument auxiliaire (6) et mobile de manière contrôlée et motorisée pour le guidage de l'instrument médical auxiliaire (20) dans l'espace d'opération (12), et ce grâce à des signaux de commande (SS) générés par une unité de commande (CU), dans lequel est exécutée, dans l'unité de commande (CU), au moins une routine de commande vocale (SSR) au moyen de laquelle les différentes instructions vocales (SB, SB1, SB2) sont enregistrées, exploitées et des signaux de commande associés (SS) sont déterminés en fonction de ceci, **caractérisé en ce que** la routine de commande vocale (SSR) est conçue pour enregistrer et exploiter des combinaisons d'instructions vocales (SBK) composées d'au moins une première et une deuxième instruction vocale (SB1, SB2), étant associée à la première instruction vocale (SB1) une information sur la direction ou la vitesse (RI, VI, RI1 à RI10) et à la deuxième instruction vocale (SB2) une information sur l'amplitude (BI, BI1 à BI10) dans un système de coordonnées de référence (BKS), et que, en fonction de l'information enregistrée sur la direction ou la vitesse (RI, VI, RI1 à RI10) et de l'information enregistrée sur l'amplitude (BI, BI1 à BI10) de la combinaison d'instructions vocales (SBK), au moins un signal de commande associé (SS) est déterminé, signal grâce auquel au moins le mouvement de l'instrument médical auxiliaire (20) est commandé au niveau de la direction ou de la vitesse et de l'amplitude, l'information sur la direction (RI, RI1 à RI10) indiquant la direction de mouvement de l'instrument auxiliaire dans le système de coordonnées cartésien de référence (BKS) et l'information sur l'amplitude (BI, BI1 à BI10) indiquant la longueur du parcours ou la trajectoire le long de la direction de mouvement dans le système de coordonnées cartésien de référence (BKS), la routine de commande vocale (SSR) étant conçue pour enregistrer et exploiter des première et deuxième instructions vocales se succédant directement (SB1, SB2) pendant une durée prédéfinie.

2. Système d'assistance chirurgical selon la revendication 1, **caractérisé en ce que** la première et la deuxième instructions vocales (SB1, SB2) comprennent un ou plusieurs mots.

3. Système d'assistance chirurgical selon la revendication 1 ou 2, **caractérisé en ce que** la commande vocale est activée au moyen de la routine de commande vocale (SSR) exclusivement en cas d'actionnement d'un élément d'activation, de préférence un interrupteur ou une touche d'activation par l'opérateur, ou en présence d'une instruction vocale d'activation.

4. Système d'assistance chirurgical selon une des revendications précédentes, **caractérisé en ce que**, au moyen d'une troisième instruction vocale, la position réelle de l'instrument auxiliaire (20) par rapport au système de coordonnées de référence (BKS) peut être sauvegardée et redémarrée de manière ciblée au moyen d'une quatrième instruction vocale.

5. Procédé de génération de signaux de commande (SS) pour la commande d'une cinématique robotique mobile par commande motorisée (3, 4, 5) d'un système d'assistance chirurgical pour le guidage d'un instrument médical auxiliaire (20), en particulier d'une caméra endoscopique, dans lequel l'instrument médical auxiliaire (20) est disposé par l'intermédiaire d'un support d'instrument auxiliaire (6) à l'extrémité libre au niveau de la cinématique robotique (3, 4, 5), dans lequel l'instrument médical auxiliaire (20) est introductible par un premier orifice chirurgical (11) du moins par sections dans l'espace d'opération (12) d'un corps de patient (10) et dans lequel les signaux de commande (SS) d'au moins une routine de commande vocale (SSR) exécutée dans une unité de commande (CU) sont déterminés, **caractérisé en ce que**, au moyen de la routine de commande vocale (SSR), une combinaison d'instructions vocales (SBK) composée d'au moins une première et une deuxième instruction vocale (SB1, SB2) est enregistrée et exploitée, étant associée à la première instruction vocale (SB1) une information sur la direction ou la vitesse (RI, VI, RI1 à RI10) et à la deuxième instruction vocale (SB2) une information sur l'amplitude (BI, BI1 à BI10) dans un système de coordonnées de référence (BKS), et que, en fonction de l'information enregistrée sur la direction ou la vitesse (RI, VI, RI1 à RI10) et de l'information enregistrée sur l'amplitude (BI, BI1 à BI10) de la combinaison d'instructions vocales (SBK), au moins un signal de commande associé (SS) est déterminé, signal grâce auquel au moins le mouvement de l'instrument médical auxiliaire (20) est commandé au niveau de la direction ou de la vitesse et de l'amplitude, l'information sur la direction (RI, RI1 à RI10) indiquant la direction de mouvement de l'instrument auxiliaire dans le système de coordonnées cartésien de référence (BKS) et l'information sur l'amplitude (BI, BI1 à BI10) indiquant la longueur du parcours ou la trajectoire le long de la direction de mouvement dans le système de coordonnées cartésien de référence (BKS), des première et deuxième instructions vocales se succédant directement (SB1, SB2) étant enregistrées et exploitées par la routine de commande vocale (SSR) pendant une durée prédéfinie.
